# EUROPEAN PATENT APPLICATION

(11) **EP 4 567 037 A1**
(43) Date of publication of application: **11.06.2025**
(21) Application number: 23849494.2
(22) Date of filing: 03.08.2023
(51) Int. Cl.: C07D 487/04, A61K 31/519, A61P 37/06

(54) **USE OF PYRROLOPYRIMIDINE COMPOUND IN TREATMENT OF ACUTE GRAFT VERSUS HOST DISEASE**

(30) Priority: 04.08.2022 CN 202210932006
(71) Applicant: Chia Tai Tianqing Pharmaceutical Group Co., Ltd., Lianyungang, Jiangsu 222062 (CN); Shouyao Holdings (Beijing) Co., Ltd., Beijing 100176 (CN); Lianyungang Runzhong Pharmaceutical Co., Ltd., Lianyungang, Jiangsu 222069 (CN)
(72) Inventor: TU, Lifan, Lianyungang, Jiangsu 222062 (CN); ZHANG, Xiquan, Lianyungang, Jiangsu 222062 (CN); WANG, Xunqiang, Lianyungang, Jiangsu 222062 (CN); YU, Ding, Lianyungang, Jiangsu 222062 (CN); LI, Chaoyi, Lianyungang, Jiangsu 222062 (CN)
(74) Representative: WSL Patentanwälte Partnerschaft mbB
(86) International application number: PCT/CN2023/110930
(87) International publication number: WO 2024/027790

(57) **Abstract**

Provided is the use of a pyrrolopyrimidine compound in the treatment of acute graft versus host disease, in particular the use of a compound of formula **I,** a stereoisomer thereof, or a pharmaceutically acceptable salt thereof in the treatment of acute graft versus host disease.

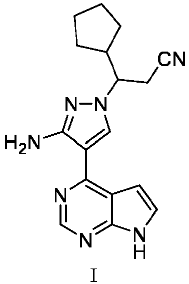

## Description

### CROSS-REFERENCE TO RELATED APPLICATION

The present application claims priority and benefit to the Chinese Patent Application No. 202210932006.7 filed with China National Intellectual Property Administration on Aug. 04, 2022, the disclosure of which is incorporated herein by reference in its entirety.

### TECHNICAL FIELD

The present application relates to the field of medicinal chemistry, and relates to use of a pyrrolopyrimidine compound for treating acute graft-versus-host disease, specifically to use of a compound of formula **I**, a stereoisomer thereof, or a pharmaceutically acceptable salt thereof for treating acute graft-versus-host disease.

### BACKGROUND

Janus kinase (JAK) is a group of non-receptor tyrosine kinases (PTKs), which exists in cells and transmits cytokine stimulation signals through the JAK-STAT pathway. The JAK-STAT pathway transmits extracellular chemical signals through the cell membrane to the gene promoter of DNA in the nucleus, which ultimately causes changes in DNA transcription and activity within the cell. The JAK-STAT pathway consists of three major components: 1) a receptor; 2) the JAK; and 3) a signal transducer and activator of transcription (STAT) protein. The receptor can be activated by interferons, interleukins, growth factors, or other chemical messengers, leading to the autophosphorylation of JAK; the STAT protein binds to the phosphorylated receptor, such that the STAT protein is phosphorylated by JAK; then the phosphorylated STAT protein is separated from the receptor, dimerized and translocated into the nucleus to bind to the specific sites on DNA and alter the transcription (Scott, M. J., C. J. Godshall, et al., (2002) "Jaks, STATs, Cytokines, and Sepsis", Clin Diagn Lab Immunol 9(6):1153-9).

Allogeneic hematopoietic stem cell transplantation (allo-HSCT) is now widely used for the treatment of hematologic and non-hematologic diseases, and is even the only method to cure certain diseases. Graft-versus-host disease (GVHD) is a major complication of allogeneic stem cell transplantation. It is due to the fact that T lymphocytes in the transplanted allogeneic donor graft are stimulated by a series of "cytokine storms" initiated by the recipient, such that the immune response to the antigen of the recipient is greatly enhanced, and cytotoxic attacks against the target cells of the recipient are triggered, with the skin, liver, and intestinal tract as the main targets. Acute graft-versus-host disease (aGVHD), also known as fulminant graft-versus-host disease, is usually symptomatic within the first 100 days after allogeneic hematopoietic stem cell transplantation. It is generally characterized by selective damage to the skin, liver, mucosa, and gastrointestinal tract.

Human and animal models have demonstrated that abnormal B-lymphocyte signaling and survival are important in the pathogenesis of GVHD. B cell targeted drugs, including SYK inhibitors (fostamatinib-Sarantopoulos et al., Biology of Blood and Marrow Transplantation, 21(2015) S11-S18) and BTK inhibitors (ibrutinib-Nakasone et al., Int. J. Hematol. Mar. 27, 2015), are shown to be capable of selectively reducing the function and frequency of abnormal GVHD B cell populations *in vitro.*

### SUMMARY

In one aspect, the present application provides a compound of formula **I,** a stereoisomer thereof, or a pharmaceutically acceptable salt thereof for use in treating graft-versus-host disease in a patient:

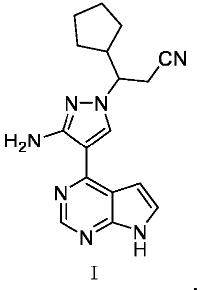

In one aspect, the present application provides a pharmaceutical composition for use in treating graft-versus-host disease in a patient, which comprises the compound of formula **I**, the stereoisomer thereof, or the pharmaceutically acceptable salt thereof.

In one aspect, the present application provides a method for treating graft-versus-host disease in a patient, which comprises administering to the patient an effective amount of the compound of formula **I**, the stereoisomer thereof, or the pharmaceutically acceptable salt thereof, or the pharmaceutical composition thereof described above.

In one aspect, the present application provides use of the compound of formula **I**, the stereoisomer thereof, or the pharmaceutically acceptable salt thereof, or the pharmaceutical composition thereof described above for preparing a medicament for treating graft-versus-host disease in a patient.

In one aspect, the present application provides use of the compound of formula **I**, the stereoisomer thereof, or the pharmaceutically acceptable salt thereof, or the pharmaceutical composition thereof described above for treating graft-versus-host disease in a patient.

In some embodiments of the present application, the graft-versus-host disease is selected from acute graft-versus-host disease.

In another aspect, the present application provides a compound of formula **I**, a stereoisomer thereof, or a pharmaceutically acceptable salt thereof for use in treating acute graft-versus-host disease in a patient:

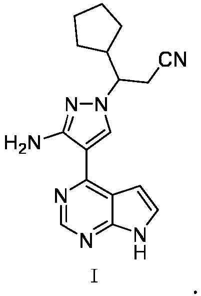

In another aspect, the present application provides a pharmaceutical composition for use in treating acute graft-versus-host disease in a patient, which comprises the compound of formula **I,** the stereoisomer thereof, or the pharmaceutically acceptable salt thereof.

In another aspect, the present application provides a method for treating acute graft-versus-host disease in a patient, which comprises administering to the patient an effective amount of the compound of formula **I,** the stereoisomer thereof, or the pharmaceutically acceptable salt thereof, or the pharmaceutical composition thereof described above.

In another aspect, the present application provides use of the compound of formula **I,** the stereoisomer thereof, or the pharmaceutically acceptable salt thereof, or the pharmaceutical composition thereof described above for preparing a medicament for treating acute graft-versus-host disease in a patient.

In another aspect, the present application provides use of the compound of formula **I,** the stereoisomer thereof, or the pharmaceutically acceptable salt thereof, or the pharmaceutical composition thereof described above for treating acute graft-versus-host disease in a patient.

In some embodiments of the present application, the compound of formula **I,** the stereoisomer thereof, or the pharmaceutically acceptable salt thereof described herein is used as a single active agent.

In some embodiments of the present application, the compound of formula **I,** the stereoisomer thereof, or the pharmaceutically acceptable salt thereof described herein may be a pharmaceutical composition comprising a therapeutically effective amount of the compound of formula **I,** the stereoisomer thereof, or the pharmaceutically acceptable salt thereof.

The pharmaceutical composition of the present application may be prepared by combining the compound of the present application with a suitable pharmaceutically acceptable excipient, and may be formulated, for example, into a solid, semisolid, liquid, or gaseous formulation such as tablet, pill, capsule, powder, granule, ointment, emulsion, suspension, suppository, injection, inhalant, gel, microsphere, and aerosol. The pharmaceutical composition of the present application may be manufactured using methods well known in the art, such as conventional mixing, dissolving, granulating, dragee-making, levigating, emulsifying, and lyophilizing. Suitable excipients include, but are not limited to: binders, diluents, wetting agents, disintegrants, lubricants, glidants, sweeteners, flavoring agents, or the like.

In some embodiments of the present application, the pharmaceutical composition is a formulation suitable for oral administration, including tablets, capsules, powders, granules, dripping pills, pastes, pulvis, and the like, preferably tablets and capsules. The oral formulation may be prepared by a conventional method using a pharmaceutically acceptable carrier well known in the art. The pharmaceutically acceptable carrier includes diluents, binders, wetting agents, disintegrants, lubricants, and the like. The diluents include microcrystalline cellulose, mannitol, lactose, sucrose, starch, pregelatinized starch, dextrin, etc., or a mixture thereof; the binders include hydroxypropyl methylcellulose, carboxymethyl cellulose, carboxymethylcellulose sodium, ethyl cellulose, methyl cellulose, hydroxypropyl cellulose, low-substituted hydroxypropyl cellulose, gelatin, polyvinylpyrrolidone, starch, sucrose, glucose, gelatin, etc., or a mixture thereof; the wetting agents include magnesium stearate, talcum powder, polyethylene glycol, sodium dodecyl sulfate, silica gel micropowder, talcum powder, etc., or a mixture thereof; the disintegrants include sodium carboxymethyl starch, dry starch, microcrystalline cellulose, hydroxyethyl methylcellulose, carboxymethylcellulose sodium, carboxymethylcellulose calcium, croscarmellose sodium, low-substituted hydroxypropyl methylcellulose, crospovidone, etc., or a mixture thereof; the lubricants include magnesium stearate, colloidal silicon dioxide, talcum powder, polyethylene glycol, stearic acid, sodium stearyl fumarate, etc., or a mixture thereof. The pharmaceutical excipients also include coloring agents, sweeteners, coating agents, and the like.

### Compound of formula I

In some embodiments of the present application, the compound of formula **I** described herein is a compound of formula **II:**

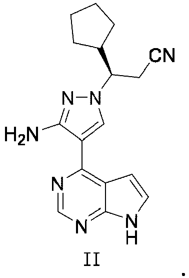

The compound of formula **I** and the compound of formula **II** of the present application may be prepared with reference to the preparation methods in Patent Nos. WO2016095805 and WO2017215627.

### Acute graft-versus-host disease (aGVHD)

In some embodiments of the present application, acute graft-versus-host disease refers to a complication caused by allogeneic stem cell transplantation.

In some embodiments of the present application, the patient has previously received allogeneic hematopoietic stem cell transplantation. In some embodiments, the allogeneic hematopoietic stem cell transplantation includes, but is not limited to: non-myeloablative, myeloablative, and reduced-intensity conditioning transplantation of bone marrow, peripheral blood stem cells, and umbilical cord blood.

In some embodiments of the present application, the acute graft-versus-host disease is classified into classical acute graft-versus-host disease, delayed acute graft-versus-host disease, persistent acute graft-versus-host disease, and recurrent acute graft-versus-host disease.

In some embodiments of the present application, the classical acute graft-versus-host disease usually occurs within 100 days after transplantation or occurs after infusion of donor's white blood cells.

In some embodiments of the present application, the persistent acute graft-versus-host disease occurs 100 days after transplantation or occurs after infusion of donor's white blood cells.

In some embodiments of the present application, the acute graft-versus-host disease may be graded into grade I, grade II, grade III, and grade IV according to the grading criteria of the Acute GVHD International Consortium (MAGIC).

In some embodiments of the present application, the acute graft-versus-host disease is graded according to the grading criteria of the aGVHD International Consortium (MAGIC); it is selected from the group consisting of grade I, grade II, grade III, and grade IV In some embodiments, it is preferably selected from the group consisting of grade II, grade III, and grade IV.

In some embodiments of the present application, organs affected by the acute graft-versus-host disease include, but are not limited to: mucosa, skin, oral cavity, eye, gastrointestinal tract, liver, lung, joint/fascia, and reproductive tract.

In some embodiments of the present application, the acute graft-versus-host disease described herein is acute graft-versus-host disease of grade I, grade II, grade III, or grade IV. In some embodiments, the acute graft-versus-host disease is acute graft-versus-host disease of grade II to grade IV. In some embodiments, the acute graft-versus-host disease is acute graft-versus-host disease of grade II to grade III. In some embodiments, the acute graft-versus-host disease is acute graft-versus-host disease of grade II to grade IV that is resistant to glucocorticoid. In some embodiments, the acute graft-versus-host disease is acute graft-versus-host disease of grade II to grade III that is resistant to glucocorticoid.

In some embodiments of the present application, the acute graft-versus-host disease is resistant to glucocorticoid therapy.

In some embodiments of the present application, the acute graft-versus-host disease is resistant to glucocorticoid therapy; the "resistance" includes disease progression, no amelioration, or incomplete response.

In some embodiments of the present application, the acute graft-versus-host disease is resistant to glucocorticoid therapy; the "resistance" includes: 1) progression of aGVHD after the glucocorticoid therapy has been administered for 3 days; and/or 2) no amelioration in aGVHD after the glucocorticoid therapy has been administered for 5 days; and/or 3) incomplete response of aGVHD after the glucocorticoid therapy has been administered for 14 days.

In some embodiments of the present application, the acute graft-versus-host disease is resistant to glucocorticoid therapy, and the resistance includes: in the case of administration of methylprednisolone at a dose of ≥ 1 mg/kg/day or another type of glucocorticoid at an equivalent dose, 1) progression of aGVHD after the glucocorticoid therapy has been administered for 3 days; and/or 2) no amelioration in aGVHD after the glucocorticoid therapy has been administered for 5 days; and/or 3) incomplete response of aGVHD after the glucocorticoid therapy has been administered for 14 days. In some embodiments of the present application, the glucocorticoid is selected from the group consisting of prednisone, meprednisone, prednisone acetate, prednisolone, methylprednisolone, prednisolone acetate, prednisolone sodium succinate, methylprednisolone sodium succinate, betamethasone, beclomethasone propionate, hydrocortisone, dexamethasone, and pulsed high-dose glucocorticoid.

In some embodiments of the present application, the acute graft-versus-host disease is acute graft-versus-host disease that is intolerant to hormonal drug therapy. In some embodiments, the acute graft-versus-host disease is acute graft-versus-host disease resistant to glucocorticoid. In some embodiments, the acute graft-versus-host disease is acute graft-versus-host disease that has not previously received hormonal drug therapy. In some embodiments, the acute graft-versus-host disease is acute graft-versus-host disease that has not previously received extracorporeal photopheresis therapy. In some embodiments, the acute graft-versus-host disease is steroid-refractory acute graft-versus-host disease.

In some embodiments of the present application, the acute graft-versus-host disease is acute graft-versus-host disease that has previously received extracorporeal photopheresis therapy. The extracorporeal photopheresis therapy mainly includes ultraviolet irradiation, wherein the ultraviolet irradiation includes, but is not limited to: ultraviolet UVA, ultraviolet UVB, ultraviolet UVC, and ultraviolet UVD, preferably ultraviolet UVA.

In some embodiments of the present application, the acute graft-versus-host disease is acute graft-versus-host disease that has previously received drug therapy. In some embodiments of the present application, the drug for the drug therapy includes, but is not limited to: one or more of hormonal drugs, calcineurin inhibitors, mammalian target of rapamycin (M-Tor) inhibitors, and immunosuppressants. The hormonal drug is an adrenocortical hormone drug, including, but not limited to: adrenocorticotropic hormone, glucocorticoid, or mineralocorticoid, preferably glucocorticoid. The hormonal drug includes, but is not limited to: prednisone, methylprednisone, prednisolone, methylprednisolone, methylprednisolone sodium succinate, betamethasone, beclomethasone propionate, diprospan, hydrocortisone, dexamethasone, or pulsed high-dose glucocorticoid. The calcineurin inhibitor includes, but is not limited to: tacrolimus or cyclosporine. The mammalian target of rapamycin (M-Tor) inhibitor includes, but is not limited to: sirolimus or tacrolimus. The immunosuppressant includes an immune antibody drug, wherein the immune antibody drug includes, but is not limited to: an anti-T cell monoclonal antibody (anti-CD3 monoclonal antibody), an anti-interleukin-2 receptor antibody, an anti-TNF antibody, and the like. The immunosuppressant includes, but is not limited to: daclizumab, basiliximab, alemtuzumab, or rituximab. The drug further includes, but is not limited to: ganciclovir sodium, imatinib, mycophenolate sodium, mycophenolate mofetil, azathioprine, psoralen, methotrexate, hydroxychloroquine, clofazimine, cyclophosphamide, thalidomide, or alefacept.

In some embodiments of the present application, the acute graft-versus-host disease is acute graft-versus-host disease resistant to glucocorticoid; the resistance to glucocorticoid includes resistance to monotherapy of glucocorticoid, or resistance to combined therapy of glucocorticoid and one or more of other drugs.

In some embodiments of the present application, the acute graft-versus-host disease is acute graft-versus-host disease resistant to monotherapy of glucocorticoid. In some embodiments of the present application, the glucocorticoid is methylprednisolone or prednisone.

In some embodiments of the present application, the acute graft-versus-host disease is acute graft-versus-host disease resistant to combined therapy of glucocorticoid and a calcineurin inhibitor. In some embodiments of the present application, the glucocorticoid is methylprednisolone or prednisone. In some embodiments of the present application, the calcineurin inhibitor is tacrolimus or cyclosporine.

In some embodiments of the present application, the acute graft-versus-host disease is acute graft-versus-host disease resistant to combined therapy of glucocorticoid, a calcineurin inhibitor, and mycophenolate mofetil. In some embodiments of the present application, the glucocorticoid is methylprednisolone or prednisone. In some embodiments of the present application, the calcineurin inhibitor is tacrolimus or cyclosporine.

In some embodiments of the present application, the acute graft-versus-host disease is acute graft-versus-host disease resistant to combined therapy of glucocorticoid, a calcineurin inhibitor, mycophenolate mofetil, and methotrexate. In some embodiments of the present application, the glucocorticoid is methylprednisolone or prednisone. In some embodiments of the present application, the calcineurin inhibitor is tacrolimus or cyclosporine.

In some embodiments of the present application, the acute graft-versus-host disease is acute graft-versus-host disease resistant to combined therapy of glucocorticoid and a CD25 monoclonal antibody. In some embodiments of the present application, the glucocorticoid is methylprednisolone or prednisone. In some embodiments of the present application, the calcineurin inhibitor is tacrolimus or cyclosporine. In some embodiments of the present application, the CD25 monoclonal antibody is basiliximab. In some embodiments of the present application, the resistance to glucocorticoid therapy includes resistance to taking high-dose systemic glucocorticoid, wherein taking the high-dose systemic glucocorticoid is taking methylprednisolone at a dose of 1-2 mg/kg/day or taking prednisone at an equivalent dose of 1.25-2.5 mg/kg/day; the methylprednisolone or prednisone is taken alone or taken in combination with a calcineurin inhibitor (CNI).

In some embodiments of the present application, within 48 h before the start of therapy for acute graft-versus-host disease, the neutrophil count (ANC) is > 1 × 10⁹/L, and the platelet count (PLT) is ≥ 20 × 10⁹/L.

In some embodiments of the present application, provided is use of the compound of formula **I,** the stereoisomer thereof, or the pharmaceutically acceptable salt thereof for preparing a medicament for acute graft-versus-host disease that has failed previous drug therapy. In some embodiments, provided is use of the compound of formula **I,** the stereoisomer thereof, or the pharmaceutically acceptable salt thereof for preparing a medicament for treating acute graft-versus-host disease resistant to hormonal drugs. In some embodiments, provided is use of the compound of formula **I,** the stereoisomer thereof, or the pharmaceutically acceptable salt thereof for preparing a medicament for treating acute graft-versus-host disease of grade II to grade IV that is resistant to hormonal drugs. In some embodiments, provided is use of the compound of formula **I,** the stereoisomer thereof, or the pharmaceutically acceptable salt thereof for preparing a medicament for treating acute graft-versus-host disease resistant to glucocorticoid. In some embodiments, provided is use of the compound of formula **I,** the stereoisomer thereof, or the pharmaceutically acceptable salt thereof for preparing a medicament for treating acute graft-versus-host disease of grade II to grade IV that is resistant to glucocorticoid.

In some embodiments, provided is use of the compound of formula **I,** the stereoisomer thereof, or the pharmaceutically acceptable salt thereof for preparing a medicament for treating acute graft-versus-host disease resistant to prednisone, meprednisone, prednisone acetate, prednisolone, methylprednisolone, prednisolone acetate, prednisolone sodium succinate, or methylprednisolone sodium succinate. In some embodiments, provided is use of the compound of formula **I,** the stereoisomer thereof, or the pharmaceutically acceptable salt thereof for preparing a medicament for treating acute graft-versus-host disease of grade II to grade IV that is resistant to prednisone, meprednisone, prednisone acetate, prednisolone, methylprednisolone, prednisolone acetate, prednisolone sodium succinate, or methylprednisolone sodium succinate. In some embodiments, provided is use of the compound of formula **I,** the stereoisomer thereof, or the pharmaceutically acceptable salt thereof for preparing a medicament for treating acute graft-versus-host disease resistant to methylprednisone or methylprednisolone. In some embodiments, provided is use of the compound of formula **I,** the stereoisomer thereof, or the pharmaceutically acceptable salt thereof for preparing a medicament for treating acute graft-versus-host disease of grade II to grade IV that is resistant to methylprednisone or methylprednisolone.

### Administration regimen

In some embodiments of the present application, the administration cycle for treating acute graft-versus-host disease in a patient is 2-6 weeks. In some embodiments of the present application, the administration cycle for treating acute graft-versus-host disease in a patient is 2 weeks, 3 weeks, 4 weeks, 5 weeks, 6 weeks, or a range formed by any of the foregoing values. In some embodiments of the present application, the administration cycle for treating acute graft-versus-host disease in a patient is 4 weeks. The amount of the compound, the stereoisomer thereof, or the pharmaceutically acceptable salt thereof disclosed herein may be determined on the basis of the severity of the disease, the response to the disease, any treatment-related toxicity, and the age and health condition of the patient. For example, it may be determined on the basis of the subject/patient's blood routine examination results, which include platelet count, neutrophil count, hemoglobin concentration, or the like. In some embodiments, a daily dose of the compound, the stereoisomer thereof, or the pharmaceutically acceptable salt thereof disclosed herein administered is 1 mg to 50 mg. In some embodiments, a daily dose of the compound, the stereoisomer thereof, or the pharmaceutically acceptable salt thereof disclosed herein administered may be selected from the group consisting of 1 mg, 5 mg, 10 mg, 15 mg, 20 mg, 25 mg, 30 mg, 35 mg, 40 mg, 45 mg, and 50 mg, and a range of any two of the foregoing values as endpoints and any value therein, for example, 1 mg to 50 mg, 5 mg to 50 mg, 5 mg to 40 mg, 5 mg to 30 mg, 5 mg to 25 mg, 5 mg to 20 mg, 10 mg to 20 mg, and the like. In some specific embodiments, a daily dose of the compound, the stereoisomer thereof, or the pharmaceutically acceptable salt thereof disclosed herein administered may be selected from the group consisting of 5 mg to 30 mg, 5 mg to 25 mg, 5 mg to 20 mg, and 10 mg to 20 mg. In some specific embodiments, a daily dose of the compound, the stereoisomer thereof, or the pharmaceutically acceptable salt thereof disclosed herein administered may be selected from the group consisting of 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg, and 50 mg, and a range of any two of the foregoing values as endpoints and any value therein, for example, 5 mg to 30 mg, 5 mg to 25 mg, 5 mg to 20 mg, 10 mg to 20 mg, and the like.

The compound, the stereoisomer thereof, or the pharmaceutically acceptable salt thereof disclosed herein may be administered once or multiple times a day. In some embodiments, the compound, the stereoisomer thereof, or the pharmaceutically acceptable salt thereof disclosed herein is administered once or twice a day. The compound, the stereoisomer thereof, or the pharmaceutically acceptable salt thereof disclosed herein may also be administered in a single dose. In one embodiment, the compound, the stereoisomer thereof, or the pharmaceutically acceptable salt thereof disclosed herein is administered in a single dose once or twice a day. In one embodiment, the compound, the stereoisomer thereof, or the pharmaceutically acceptable salt thereof disclosed herein is administered in the form of a single-dose oral solid formulation once or twice a day. In one specific embodiment, the compound, the stereoisomer thereof, or the pharmaceutically acceptable salt thereof disclosed herein is administered in the form of a single-dose oral solid formulation twice a day.

The compound, the stereoisomer thereof, or the pharmaceutically acceptable salt thereof disclosed herein may also be administered in multiple doses. In one embodiment, the compound, the stereoisomer thereof, or the pharmaceutically acceptable salt thereof disclosed herein is administered in multiple doses once or twice a day. In one embodiment, the compound, the stereoisomer thereof, or the pharmaceutically acceptable salt thereof disclosed herein is administered in the form of a multiple-dose oral solid formulation once or twice a day. In one specific embodiment, the compound, the stereoisomer thereof, or the pharmaceutically acceptable salt thereof disclosed herein is administered in the form of a multiple-dose oral solid formulation twice a day.

In some embodiments of the present application, the compound of formula **I,** the stereoisomer thereof, or the pharmaceutically acceptable salt thereof is provided in the form of a pharmaceutical composition, preferably a single-dose pharmaceutical composition. In some embodiments, the pharmaceutical composition comprises 1 mg to 50 mg of the compound, the stereoisomer thereof, or the pharmaceutically acceptable salt thereof disclosed herein. In some embodiments, the pharmaceutical composition comprises 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg, or 50 mg, or a range of any two of the foregoing values as endpoints or any value therein of the compound, the stereoisomer thereof, or the pharmaceutically acceptable salt thereof disclosed herein, for example, 5 mg to 30 mg, 5 mg to 25 mg, 5 mg to 20 mg, 10 mg to 20 mg, or the like. In some embodiments, the pharmaceutical composition comprises 5 mg, 10 mg, 15 mg, or 20 mg of the compound, the stereoisomer thereof, or the pharmaceutically acceptable salt thereof disclosed herein. In some embodiments, the pharmaceutical composition comprises 10 mg or 15 mg of the compound, the stereoisomer thereof, or the pharmaceutically acceptable salt thereof disclosed herein. In some embodiments, the pharmaceutical composition comprises 10 mg of the compound, the stereoisomer thereof, or the pharmaceutically acceptable salt thereof disclosed herein.

In some embodiments of the present application, the pharmaceutical composition of the compound of formula **I,** the stereoisomer thereof, or the pharmaceutically acceptable salt thereof is in a daily dose. In some embodiments of the present application, the pharmaceutical composition of the compound of formula **I,** the stereoisomer thereof, or the pharmaceutically acceptable salt thereof is in twice-daily doses. In some embodiments of the present application, the twice-daily doses are in the same dose.

In some embodiments of the present application, the pharmaceutical composition of the compound of formula **I,** the stereoisomer thereof, or the pharmaceutically acceptable salt thereof is in twice-daily doses, and the dose for each administration is in a single dose or multiple doses.

In some embodiments of the present application, the pharmaceutical composition of the compound of formula **I,** the stereoisomer thereof, or the pharmaceutically acceptable salt thereof is in twice-daily doses, and the dose for each administration is in multiple doses consisting of single doses of 5 mg, 10 mg, 15 mg, and/or 20 mg of the compound of formula **I,** the stereoisomer thereof, or the pharmaceutically acceptable salt thereof.

In some embodiments of the present application, the pharmaceutical composition consists of single doses of 5 mg of the compound of formula **I,** the stereoisomer thereof, or the pharmaceutically acceptable salt thereof.

In some embodiments of the present application, the pharmaceutical composition of the compound of formula **I,** the stereoisomer thereof, or the pharmaceutically acceptable salt thereof is in twice-daily doses, and the dose for each administration is in multiple doses consisting of single doses of 5 mg of the compound of formula **I,** the stereoisomer thereof, or the pharmaceutically acceptable salt thereof; the dose for each administration is 5 mg, 10 mg, 15 mg, or 20 mg, or the dose for each administration is 10 mg or 15 mg, or the dose for each administration is 10 mg.

In some embodiments of the present application, the pharmaceutical composition of the compound of formula **I,** the stereoisomer thereof, or the pharmaceutically acceptable salt thereof is in twice-daily doses, and the dose for each administration is in a single dose of 5 mg, 10 mg, 15 mg, and/or 20 mg of the compound of formula **I,** the stereoisomer thereof, or the pharmaceutically acceptable salt thereof. In some embodiments of the present application, the single dose is 20 mg of the compound of formula **I,** the stereoisomer thereof, or the pharmaceutically acceptable salt thereof.

In some embodiments of the present application, the pharmaceutical composition of the compound of formula **I,** the stereoisomer thereof, or the pharmaceutically acceptable salt thereof is packaged in a kit, which also contains instructions for use of the compound of formula **I,** the stereoisomer thereof, or the pharmaceutically acceptable salt thereof for treating acute graft-versus-host disease.

In some embodiments of the present application, in the method or use for treating acute graft-versus-host disease, the pharmaceutical composition of the compound of formula **I,** the stereoisomer thereof, or the pharmaceutically acceptable salt thereof is in a daily dose and is administered as follows: the pharmaceutical composition of the compound of formula **I,** the stereoisomer thereof, or the pharmaceutically acceptable salt thereof is administered once or twice a day. In some embodiments of the present application, the pharmaceutical composition of the compound of formula **I,** the stereoisomer thereof, or the pharmaceutically acceptable salt thereof is administered twice a day in the same dose. In some embodiments of the present application, the pharmaceutical composition of the compound of formula **I,** the stereoisomer thereof, or the pharmaceutically acceptable salt thereof is administered twice a day in the same dose at an interval of 12 h.

In some embodiments of the present application, 28 days constitute one treatment cycle, in which the pharmaceutical composition of the compound of formula **I,** the stereoisomer thereof, or the pharmaceutically acceptable salt thereof is administered consecutively from day 1 to day 28.

In some embodiments of the present application, 28 days constitute one treatment cycle, in which the pharmaceutical composition of the compound of formula **I,** the stereoisomer thereof, or the pharmaceutically acceptable salt thereof is administered twice a day consecutively from day 1 to day 28.

In some embodiments of the present application, 28 days constitute one treatment cycle, in which the pharmaceutical composition of the compound of formula **I,** the stereoisomer thereof, or the pharmaceutically acceptable salt thereof is administered twice a day in the same dose consecutively from day 1 to day 28.

In some embodiments of the present application, 28 days constitute one treatment cycle, in which the pharmaceutical composition of the compound of formula **I,** the stereoisomer thereof, or the pharmaceutically acceptable salt thereof is administered twice a day in the same dose consecutively from day 1 to day 28, and the dose for each administration is in multiple doses consisting of single doses of 5 mg of the compound of formula **I,** the stereoisomer thereof, or the pharmaceutically acceptable salt thereof; the dose for each administration is 5 mg, 10 mg, 15 mg, or 20 mg, or the dose for each administration is 10 mg or 15 mg, or the dose for each administration is 10 mg.

In some embodiments of the present application, 28 days constitute one treatment cycle, in which a total dose of 140-840 mg of the pharmaceutical composition of the compound of formula **I**, the stereoisomer thereof, or the pharmaceutically acceptable salt thereof (calculated based on the active ingredient, the compound of formula (**I**)) is administered. In some embodiments of the present application, the total dose of the pharmaceutical composition of the compound of formula **I,** the stereoisomer thereof, or the pharmaceutically acceptable salt thereof is selected from the group consisting of 140 mg, 280 mg, 420 mg, 560 mg, 700 mg, 840 mg, and a range formed by any two of the foregoing values (calculated based on the active ingredient, the compound of formula **(I)).** In some embodiments of the present application, the total dose of the pharmaceutical composition of the compound of formula **I,** the stereoisomer thereof, or the pharmaceutically acceptable salt thereof is preferably 280-840 mg (calculated based on the active ingredient, the compound of formula **(I)).** In some embodiments of the present application, the total dose of the pharmaceutical composition of the compound of formula **I,** the stereoisomer thereof, or the pharmaceutically acceptable salt thereof is preferably 560 mg or 840 mg (calculated based on the active ingredient, the compound of formula **(I)).** In some embodiments of the present application, the total dose of the pharmaceutical composition of the compound of formula **I,** the stereoisomer thereof, or the pharmaceutically acceptable salt thereof is preferably 560 mg (calculated based on the active ingredient, the compound of formula **(I)).**

In some embodiments of the present application, the treatment cycle is repeated as long as the disease is still under control and the administration regimen is clinically tolerable. The compound, the stereoisomer thereof, or the pharmaceutically acceptable salt thereof disclosed herein may be administered by various routes, including, but not limited to: oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, and intrathecal administrations. In one specific embodiment, the route is oral administration.

In some embodiments of the present application, the pharmaceutical composition of the compound of formula **I,** the stereoisomer thereof, or the pharmaceutically acceptable salt thereof may be formulated in a form suitable for oral administration to a human, for example, including, but not limited to: tablet, pill, capsule, powder, granule, or the like.

In some embodiments of the present application, the pharmaceutical composition of the compound of formula **I,** the stereoisomer thereof, or the pharmaceutically acceptable salt thereof is in the form of an oral tablet.

In some embodiments of the present application, a single dose of the oral tablet of the compound of formula **I,** the stereoisomer thereof, or the pharmaceutically acceptable salt thereof is 5 mg or 20 mg.

In some embodiments of the present application, a single dose of the oral tablet of the compound of formula **I,** the stereoisomer thereof, or the pharmaceutically acceptable salt thereof is 5 mg.

The administration regimen described herein is also suitable for graft-versus-host disease.

### Technical effects

The compound of formula **I,** the stereoisomer thereof, or the pharmaceutically acceptable salt thereof, or the pharmaceutical composition thereof disclosed herein has favorable therapeutic effects, including, but not limited to: better objective response rate (for example, 28-day objective response rate), complete response rate (for example, 28-day complete response rate) and overall survival, longer duration of response (DOR), higher event-free survival rate, and lower non-relapse mortality and incidence of malignancy relapse/progression events. The compound of formula **I,** the stereoisomer thereof, or the pharmaceutically acceptable salt thereof, or the pharmaceutical composition thereof disclosed herein also has favorable safety in addition to favorable therapeutic effects, including, but not limited to: a lower incidence of adverse events.

### Definitions and description

Unless otherwise stated, the terms used herein shall have the following meanings. A particular term, unless otherwise specifically defined, should not be considered uncertain or unclear, but construed according to its common meaning in the art. When referring to a trade name herein, it is intended to refer to its corresponding commercial product or its active ingredient.

As used herein, unless otherwise stated, the terms "comprise", "comprises", "comprising", or equivalents thereof are open-ended statements and mean that elements, components, and steps that are not specified may be encompassed in addition to those listed.

All patents, patent applications, and other identified publications are explicitly incorporated herein by reference for the purpose of description and disclosure. Any reference to these publications herein shall not be construed as an admission that the publications form part of the commonly recognized knowledge in the art.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids and salts formed from acidic radicals and free bases. The pharmaceutically acceptable salt described herein is selected from the group consisting of a maleate salt, a hydrochloride salt, a hydrobromide salt, a sulfate salt, a phosphate salt, a nitrate salt, an acetate salt, a lactate salt, a malonate salt, a succinate salt, a fumarate salt, a malate salt, a mandelate salt, a tartrate salt, a citrate salt, an ascorbate salt, a palmitate salt, a benzoate salt, a phenylacetate salt, a cinnamate salt, a salicylate salt, a methanesulfonate salt, a benzenesulfonate salt, or a methylbenzenesulfonate salt. As used herein, the amount of the compound of formula **I,** for example, the amount administered, the dose, or the amount in the pharmaceutical composition, is calculated based on its free base form.

As used herein, if the compound in the pharmaceutical combination of the present application has, for example, at least one basic site, it may form an acid addition salt. If needed, it may further form a corresponding acid addition salt with additionally present basic sites. A compound with at least one acidic group (e.g., -COOH) may further form a salt with a base. If the compound comprises, for example, both carboxyl and amino, it may further form a corresponding inner salt.

The compound of the present application may be asymmetrical, for example, having one or more stereoisomers. Unless otherwise stated, all stereoisomers are included, for example, enantiomers and diastereoisomers. The compound of the present application containing asymmetrical carbon atoms may be separated in an optically pure form or in a racemic form. The optically pure form may be separated from a racemic mixture or may be synthesized using a chiral raw material or a chiral reagent.

The term "patient" refers to a mammal, such as a human, dog, cow, horse, pig, sheep, goat, cat, mouse, rabbit, rat, or transgenic non-human animal. In some embodiments, the patient is a human.

The term "pharmaceutical composition" refers to a mixture consisting of one or more of the compounds or the pharmaceutical combinations thereof or the salts thereof of the present application and a pharmaceutically acceptable excipient. The pharmaceutical composition is intended to facilitate the administration of the compound or the pharmaceutical combination thereof of the present application to a subject.

The term "treatment" generally refers to obtaining desired pharmacological and/or physiological effects, including partially or completely stabilizing or curing a disease and/or an effect caused by the disease. As used herein, "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "effective amount" refers to an amount of the compound of the present application used for (i) treating a specific disease, condition, or disorder, or (ii) relieving, ameliorating, or eliminating one or more symptoms of a specific disease, condition, or disorder. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The term "single dose" refers to the smallest unit of packaging containing a certain quantity of pharmaceutical product; for example, in a box of seven capsules, each capsule is a single dose; or a vial of injection is a single dose.

The term "multiple doses" consists of multiple single doses.

The terms "administer", "administration", and "administering" refer to physically introducing the composition comprising a therapeutic agent to an individual using any one of a variety of methods and delivery systems known to those skilled in the art. In certain embodiments, the administration is oral administration.

The term "daily dose" refers to a dose administered to a patient per day.

The terms "day", "daily", and the like, when referred to in an administration regimen, refer to the time within a calendar day that starts at midnight and ends at the next midnight.

As used herein, the acute graft-versus-host disease is graded according to the grading criteria of the aGVHD International Consortium (MAGIC).

As used herein, the Harris 2016 response evaluation criteria were used to evaluate disease state or efficacy.

### DETAILED DESCRIPTION

The present invention is illustrated in more detail through specific examples. The following examples are provided for illustrative purposes only and are not intended to limit the present invention in any way.

### Preparation Example 1: Preparation of Solid Pharmaceutical Composition in 5 mg and 20 mg Tablets

The formula for a solid pharmaceutical composition in 5 mg and 20 mg tablets is shown in Table 1:

**Table 1. Formula for 5 mg and 20 mg tablets**

| **Composition** | **Formula (mg)** | |
|---|---|---|
| Compound of formula **II** | 5 | 20 |
| Mannitol | 35.275 | 141.1 |
| Microcrystalline cellulose | 70 | 280 |
| Croscarmellose sodium | 3.6 | 14.4 |
| Sodium dodecyl sulfate | 0.125 | 0.5 |
| Hydroxypropyl cellulose | 4.8 | 19.2 |
| Magnesium stearate | 1.2 | 4.8 |
| Purified water | Proper amount | Proper amount |

### Procedures:

1) Mannitol, microcrystalline cellulose, and croscarmellose sodium were mixed to prepare a mixture A for further use.
   Preparation of a drug substance suspension: Hydroxypropyl cellulose was dissolved in the prescribed amount of purified water to prepare a 4% (w/w) hydroxypropyl cellulose solution; sodium dodecyl sulfate was dissolved; the compound of formula **II** was added and dispersed by stirring to prepare the drug substance suspension.
2) Fluidized bed granulation and drying: The drug substance suspension was applied to the mixture A by spraying for fluidized granulation. Granulation parameters: inlet air temperature: 55-80 °C, atomizing pressure: 600-1000 mbar, material temperature: 25-35 °C. Drying started after the spraying, and ended when the material temperature was higher than 45 °C. The materials were sized in a grinding and granulate machine through a sieve with a mesh size of Φ 0.6-1.2 mm, and dried granules after sizing were obtained.
3) The dried granules after sizing and magnesium stearate were fed into a hopper blender in sequence and well mixed to give a solid pharmaceutical composition for tableting.

### Example 1 Clinical Protocol for Acute Graft-Versus-Host Disease

This study was conducted to evaluate the safety and preliminary efficacy of the compound of formula II for treating acute graft-versus-host disease in a subject resistant to glucocorticoid.

### 1.1 Administration regimen

Administration method: orally administered twice a day at fasting, at a dose of 10 mg or 15 mg each time, with 28 consecutive days of administration as one treatment cycle.

Drug: the compound of formula **I** in 5 mg or 20 mg tablets, with the compound of formula I being shown as its stereoisomer, a compound of formula **II:**

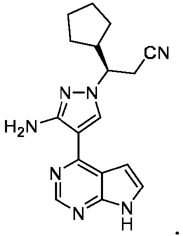

### 1.2 Enrollment criteria

1) Aged 12-75 years (inclusive), with no gender restriction;
2) Previously received allogeneic hematopoietic stem cell transplantation (including non-myeloablative, myeloablative, and reduced-intensity conditioning transplantation of bone marrow, peripheral blood stem cells, and umbilical cord blood);
3) Clinically diagnosed with aGVHD of grade II to grade IV according to MAGIC guidelines;
   Resistance to glucocorticoid therapy *, defined as resistance to high-dose systemic glucocorticoid (methylprednisolone at a dose of 1-2 mg/kg/day or prednisone at an equivalent dose of 1.25-2.5 mg/kg/day), taken alone or taken in combination with a calcineurin inhibitor (CNI);
   * Subjects meeting any one of the following conditions can be defined as resistant to conventional glucocorticoid therapy, i.e., therapy with methylprednisolone at a dose of ≥ 1 mg/kg/day or therapy with another type of glucocorticoid at an equivalent dose: (1) progression of aGVHD after the glucocorticoid therapy has been administered for 3 days, (2) no amelioration in aGVHD after the glucocorticoid therapy has been administered for 5 days, and (3) incomplete response of aGVHD after the glucocorticoid therapy has been administered for 14 days;
4) Within 48 h before the start of therapy, neutrophil count (ANC) > 1 × 10⁹/L, and platelet count (PLT) ≥ 20×10⁹/L; and
5) Subjects with voluntary participation, signed informed consent, and good compliance.

### 1.3. Evaluation method

### Evaluation of aGVHD

During the study, the investigator scored aGVHD treatment responses according to Harris 2016 response evaluation criteria.

Changes in aGVHD, including hormones, CNI use, dose adjustment of tablets of the compound of formula I, etc., needed to be monitored during the study.

### Evaluation of cGVHD

During the study, the investigator evaluated cGVHD according to NIH consensus guidelines for cGVHD once every 28 days from day 1 to day 56, evaluated cGVHD once at each visit in subsequent cycles, and made records accordingly.

### Evaluation of transplantation failure

During the study, the investigator evaluated transplantation failure. Considering that the transplantation failure is defined as donor chimerism of whole blood or bone marrow being ≥ 5% and dropping to < 5% in subsequent detection, it was necessary to perform chimerism detection on time.

If a subject experiences transplantation failure, the investigator should take any timely measures to address it, including rapidly reducing immunosuppression, administering DLI, using stem cells and/or chemotherapy, or implementing any other measures. Chimerism detection: Donor chimerism detection after hematopoietic stem cell transplantation involved identifying the genetic characteristics of a recipient and a donor before the transplantation, and then evaluating the ratio of donor cells to recipient cells in the recipient's blood or bone marrow. Chimerism detection was performed using peripheral blood mononuclear cells or bone marrow during the screening period, on day 14, day 28, day 42 and day 56, and at subsequent visits. If chimerism detection was performed within 28 days before the first administration, it will be considered as screening-period detection and will not be repeated.

### Evaluation on relapse/progression of primary malignant hematologic disease

During the study, the investigator evaluated whether the subjects' primary malignant hematologic disease had relapsed/progressed according to the visit plan, and recorded whether any therapies had been taken to treat the hematologic malignancy, including termination of immunosuppressive therapy, chemotherapy, and/or lymphocyte infusion.

### 1.4 Evaluation index

The evaluation index may be selected from the group consisting of:
Objective response rate (ORR): refers to the percentage of subjects in complete response (CR) or very good partial response (VGPR) or partial response (PR).
Complete response (CR) rate: refers to the percentage of subjects in complete response (CR).
Overall survival (OS): refers to the time from the start of the first administration to death due to any cause.
Duration of response (DOR): for subjects in complete response (CR) or very good partial response (VGPR) or partial response (PR) on day 28, DOR is defined as the time from the date of the first recorded response to the date of the first recorded disease progression or the start of any new systemic therapy for aGVHD.
Event-free survival (EFS): defined as the time from the date of the first administration to the date of relapse/progression of hematologic disease, transplantation failure, or death due to any cause.
Non-relapse mortality (NRM): defined as the time from the date of the first administration to the date of death not related to relapse/progression of hematologic disease.
Incidence of malignancy relapse/progression (MR): defined as the time from the date of the first administration to the date of relapse/progression of hematologic disease.

### 1.5 Effect data

### 1.5.1 Efficacy

Twelve evaluable subjects orally took tablets of the compound of formula II twice a day at fasting, at a dose of 10 mg each time, with 28 days as one administration cycle. The ORR of the 12 subjects on D28 was 83.3% (10/12), with 8 achieving CR and 2 achieving VGPR (very good partial response). In terms of safety, tablets of the compound of formula II had good tolerability and safety, with overall adverse events being controllable. In conclusion, tablets of the compound of formula **II** have significant clinical benefits for treating glucocorticoid-resistant acute graft-versus-host disease. Representative cases are shown in Table 2 below:

**Table 2. Evaluation of efficacy in patients**

| **Baseline characteristics** | **Number of patients (N = 12)** | **%** | **Number of patients achieving ORR on D28** |
|---|---|---|---|
| **History of previous therapy** | | | |
| Hormone ± CNI | 4 | 33.3 | 4 |
| Hormone + CNI + MMF | 6 | 50.0 | 5 |
| Hormone + MTX + CNI + MMF | 1 | 8.3 | 1 |
| Hormone + CD25 monoclonal antibody | 1 | 8.3 | 0 |

| **Grades of aGVHD** | | | |
|---|---|---|---|
| Grade II | 6 | 50.0 | 6 |
| Grade III | 6 | 50.0 | 4 |

| **Organs affected by aGVHD** | | | |
|---|---|---|---|
| Skin | 8 | 66.7 | 8 |
| Liver | 5 | 50.0 | 3 |
| Upper digestive tract | 2 | 16.7 | 2 |
| Lower digestive tract | 5 | 50.0 | 4 |
| **ORR on D28** | 10/12 | 83.3 | |

| | | | |
|---|---|---|---|
| Note: In Table 2, "Hormone" represents glucocorticoid; "CNI" represents a calcineurin inhibitor, mainly including tacrolimus and/or cyclosporine; "MMF" represents mycophenolate mofetil; "MTX" represents methotrexate; "CD25 monoclonal antibody" includes basiliximab; "Hormone ± CNI" represents administration of hormone alone or in combination with CNI. | | | |

Those skilled in the art will recognize that the scope of the present application is not limited to the embodiments and examples described above. Instead, various modifications, substitutions, or recombinations may be made without departing from the spirit of the present application, all of which fall within the protection scope of the present application.

## Claims

1. A compound of formula **I,** a stereoisomer thereof, or a pharmaceutically acceptable salt thereof for use in treating graft-versus-host disease in a patient:

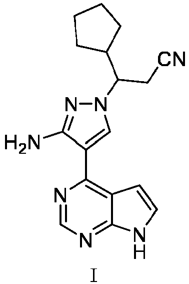

2. A pharmaceutical composition for use in treating graft-versus-host disease in a patient, comprising the compound of formula **I,** the stereoisomer thereof, or the pharmaceutically acceptable salt thereof according to claim 1.

3. Use of the compound of formula **I,** the stereoisomer thereof, or the pharmaceutically acceptable salt thereof according to claim 1, or the pharmaceutical composition according to claim 2 for preparing a medicament for treating graft-versus-host disease in a patient.

4. The compound of formula **I,** the stereoisomer thereof, or the pharmaceutically acceptable salt thereof according to claim 1, or the pharmaceutical composition according to claim 2, or the use according to claim 3, wherein the graft-versus-host disease is selected from acute graft-versus-host disease.

5. The use according to claim 3 or 4, wherein the acute graft-versus-host disease is acute graft-versus-host disease of grade I, grade II, grade III, or grade IV.

6. The use according to any one of claims 3-5, wherein the acute graft-versus-host disease is resistant to glucocorticoid therapy.

7. The use according to claim 6, wherein the glucocorticoid is selected from the group consisting of prednisone, meprednisone, prednisone acetate, prednisolone, methylprednisolone, prednisolone acetate, prednisolone sodium succinate, methylprednisolone sodium succinate, betamethasone, beclomethasone propionate, hydrocortisone, dexamethasone, and pulsed high-dose glucocorticoid.

8. The use according to any one of claims 3-7, wherein a daily dose of the compound of formula I, the stereoisomer thereof, or the pharmaceutically acceptable salt thereof is 1 mg to 50 mg; or a daily dose of the compound of formula I, the stereoisomer thereof, or the pharmaceutically acceptable salt thereof is 1 mg, 5 mg, 10 mg, 15 mg, 20 mg, 25 mg, 30 mg, 35 mg, 40 mg, 45 mg, or 50 mg, or a range of any two of the foregoing values as endpoints or any value therein, for example, 1 mg to 50 mg, 5 mg to 50 mg, 5 mg to 40 mg, 5 mg to 30 mg, 5 mg to 25 mg, 5 mg to 20 mg, or 10 mg to 20 mg; or a daily dose of the compound of formula I, the stereoisomer thereof, or the pharmaceutically acceptable salt thereof is 5 mg to 30 mg, 5 mg to 25 mg, 5 mg to 20 mg, or 10 mg to 20 mg.

9. The use according to any one of claims 3-7, wherein the pharmaceutical composition of the compound of formula **I,** the stereoisomer thereof, or the pharmaceutically acceptable salt thereof is in a daily dose; or the pharmaceutical composition of the compound of formula **I,** the stereoisomer thereof, or the pharmaceutically acceptable salt thereof is in twice-daily doses.

10. The use according to any one of claims 3-7, wherein the pharmaceutical composition of the compound of formula **I,** the stereoisomer thereof, or the pharmaceutically acceptable salt thereof is administered in multiple doses, or administered in multiple doses once or twice a day, or administered in the form of a multiple-dose oral solid formulation twice a day.

11. The use according to any one of claims 3-7, wherein the pharmaceutical composition of the compound of formula **I,** the stereoisomer thereof, or the pharmaceutically acceptable salt thereof is in twice-daily doses, and the dose for each administration is in multiple doses consisting of single doses of 5 mg of the compound of formula **I,** the stereoisomer thereof, or the pharmaceutically acceptable salt thereof; the dose for each administration is 5 mg, 10 mg, 15 mg, or 20 mg, or the dose for each administration is 10 mg or 15 mg, or the dose for each administration is 10 mg.

12. The use according to any one of claims 3-11, wherein 28 days constitute one treatment cycle, in which the pharmaceutical composition of the compound of formula **I,** the stereoisomer thereof, or the pharmaceutically acceptable salt thereof is administered twice a day consecutively from day 1 to day 28.

13. The use according to any one of claims 3-12, wherein 28 days constitute one treatment cycle, in which the pharmaceutical composition of the compound of formula **I,** the stereoisomer thereof, or the pharmaceutically acceptable salt thereof is administered twice a day in the same dose consecutively from day 1 to day 28, and the dose for each administration is in multiple doses consisting of single doses of 5 mg of the compound of formula **I,** the stereoisomer thereof, or the pharmaceutically acceptable salt thereof; the dose for each administration is 5 mg, 10 mg, 15 mg, or 20 mg, or the dose for each administration is 10 mg or 15 mg, or the dose for each administration is 10 mg.

14. The use according to any one of claims 3-13, wherein a total dose of the pharmaceutical composition of the compound of formula **I,** the stereoisomer thereof, or the pharmaceutically acceptable salt thereof is 140 mg to 840 mg, or is 280 mg to 840 mg, or is 560 mg or 840 mg, or is 560 mg.

15. The use according to any one of claims 3-14, wherein a single dose of an oral tablet of the compound of formula **I,** the stereoisomer thereof, or the pharmaceutically acceptable salt thereof is 5 mg or 20 mg, or is 5 mg.
